(19) Europäisches Patentamt
European Patent Office
Office européen des brevets

(11) **EP 2 716 225 A1**

(12) **EUROPEAN PATENT APPLICATION**
published in accordance with Art. 153(4) EPC

(43) Date of publication:
**09.04.2014 Bulletin 2014/15**

(51) Int Cl.:
***A61B 6/03*** *(2006.01)*          ***A61B 6/00*** *(2006.01)*
***G06T 1/00*** *(2006.01)*

(21) Application number: **12789201.6**

(22) Date of filing: **21.05.2012**

(86) International application number:
**PCT/JP2012/062892**

(87) International publication number:
**WO 2012/161149 (29.11.2012 Gazette 2012/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.05.2011   JP 2011116145**

(71) Applicant: **Hitachi, Ltd.**
**Tokyo 100-8280 (JP)**

(72) Inventors:
  • **MIYAKOSHI Junichi**
    **Chiyoda-ku**
    **Tokyo 100-8280 (JP)**
  • **YUI Shuntaro**
    **Chiyoda-ku**
    **Tokyo 100-8280 (JP)**
  • **MATSUZAKI Kazuki**
    **Chiyoda-ku**
    **Tokyo 100-8280 (JP)**

(74) Representative: **Strehl Schübel-Hopf & Partner**
**Maximilianstrasse 54**
**80538 München (DE)**

(54) **IMAGE PROCESSING APPARATUS AND METHOD**

(57)    The present invention relates to a technology for extracting a target area from within an image by a combination of a plurality of image processes, enabling an automatic setting of an image process procedure without an operator inputting the image process procedure. Thus, according to the present invention, the content of an image process to be carried out in the next and subsequent rounds is automatically determined based on a history of results of image processes that have been applied to a process object image up to the immediately preceding round.

Fig. 1

EP 2 716 225 A1

**Description**

TECHNICAL FIELD

The present invention relates to a technology for automatically setting a procedure for extracting a target area from within an image by a combination of a plurality of image processes.

BACKGROUND ART

The progress in diagnostic imaging apparatuses and the like has resulted in significant increases in medical images and medical information. As a result, huge volumes of medical images and medical information are being accumulated. Meanwhile, the increase in stored volumes has also led to an increased burden on clinicians and radiologists who use medical images for diagnosis. This has resulted in a situation in which the accumulated medical images and medical information are not fully utilized.

In order to effectively utilize medical images and increase the quality of diagnosis or treatment, a method for determining a plurality of image processes to be applied to a single medical image and a procedure for implementing the processes in advance has been proposed (see Patent Document 1, for example).

The document discloses an apparatus by which an analysis protocol (image analyzing procedure) to be applied to image data from a diagnostic imaging apparatus (such as a computed tomography (CT) apparatus) is determined in accordance with the purpose of examination and the examined region, and a desired processing result is obtained through an image process using parameters acquired by preprocessing. Specifically, the document discloses a technique for selecting an image process implementing procedure in advance based on image data and image-accompanying information, and for carrying out the procedure in sequence.

PRIOR ART DOCUMENT

PATENT DOCUMENT

Patent Document 1: JP Patent Publication (Kokai) No. 2009-82452 A1

SUMMARY OF THE INVENTION

PROBLEM TO BE SOLVED BY THE INVENTION

In the case of the apparatus according to the above document, prior to starting an image process (image analysis), the order of implementation of the process is automatically determined in advance. Namely, the implementation order is fixed in advance. Thus, when the content of the process is desired to be modified in the image process, the user needs to input an instruction for each change in process content. Particularly, when a desired processing result is not obtained by the image process currently being carried out, it may become necessary to change the subsequent process content.

However, if the separate operation inputs by the user are required, the burden on the user cannot be reduced.

Based on a detailed analysis of the above problem, the present inventors provide a mechanism such that the content of image processes to be sequentially applied to a process object image can be automatically determined.

MEANS FOR SOLVING THE PROBLEM

According to the present invention, the content of an image process to be carried out in the next and subsequent rounds is automatically determined based on a history of results of image processes applied to the process object image up to the immediately preceding round.

EFFECTS OF THE INVENTION

According to the present invention, the content of an image process for the next and subsequent rounds can be automatically determined by referring to the history of image process results that are stored in large volumes. Thus, the operational burden on the user when extracting a target area from the process object image through an image process can be decreased.

Other problems, configurations, and effects will become apparent from a reading of the following description of embodiments.

BRIEF DESCRIPTION OF THE DRAWINGS

FIG. 1 is a functional block diagram of an image processing system according to a first embodiment.
FIG. 2 illustrates an example of a display screen provided through an image processing apparatus according to the first embodiment.
FIG. 3 is a flowchart of a process procedure carried out by the image processing apparatus according to the first embodiment.
FIG. 4 illustrates a relationship between target area feature quantities and procedure feature quantities according to the first embodiment.
FIG. 5 illustrates the content of processing by a next-process determination unit according to the first embodiment.
FIG. 6 is a functional block diagram of the image processing system according to a second embodiment.
FIG. 7 is a flowchart of a process procedure carried out by the image processing apparatus according to the second embodiment.
FIG. 8 illustrates the content of processing by the next-process determination unit according to a third embodiment.
FIG. 9 illustrates the relationship between the target area feature quantities and the procedure feature quantities according to a fourth embodiment.

MODE FOR CARRYING OUT THE INVENTION

In the following, embodiments of the present invention will be described with reference to the drawings. The mode for carrying out the present invention is not limited to the following embodiments, and various modifications may be made within the technical scope of the present invention.

(1) Common configuration

The image processing apparatuses described below are all based on the assumption that a plurality of image processes is applied in sequence in order to extract a target area from a process object image. The image processing apparatuses according to the various embodiments are common in that a database is searched for an image process procedure with a history similar to the history of processing results acquired up to the immediately preceding round, and the content of an image process to be applied next is automatically determined from the search result. Specifically, the image processing apparatuses statistically determine the image process to be applied next based on a large amount of information about the image process procedures used in the past that are stored in the database.

In the database, the results of successive judgments made by technical experts based on experience or processing results and the like are stored as the image process procedures. Thus, it is statistically meaningful for target area extraction to search for the past image process procedure with a history similar to the history of processing results with respect to the process object image that is currently being processed, and to apply the image process for the next round used in the detected image process procedure for the current process as is. In the image processing apparatuses according to the embodiments, this determination process is repeated to automatically extract the target areas from the process object image.

(2) First embodiment

(2-1) System configuration

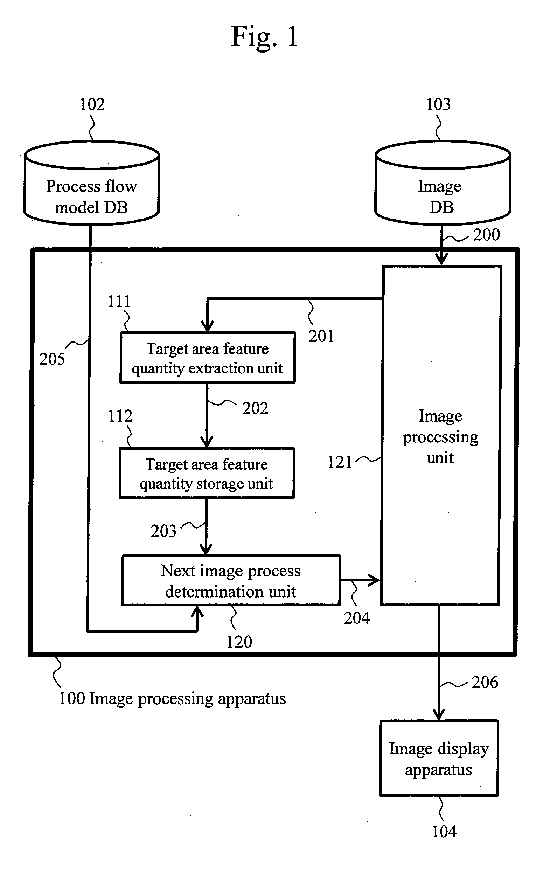

FIG. 1 is a functional block diagram of an image processing system according to the first embodiment. The image processing system according to the first embodiment includes an image processing apparatus 100, a process flow model database 102, an image database 103, and an image display apparatus 104.

In the process flow model database 102, image process procedures carried out in the past and image process procedures registered as standard models are stored. In the present specification, a "procedure" refers to information specifying an implementation order of a plurality of image processes. According to the present embodiment, the image process procedure includes a history (which may hereafter be referred to as "procedure feature quantities") of processing results (which may hereafter be referred to as "target area feature quantities") obtained upon carrying out each image process.

In the image database 103, image data as a process object are stored. According to the present embodiment, medical image data are stored. For example, contrast enhanced CT data are stored. Of course, the image data are not limited to contrast enhanced CT data.

The image processing apparatus 100 includes an image processing unit 121, a target area feature quantity extraction unit 111, a target area feature quantity storage unit 112, and a next image process determination unit 120. According to the present embodiment, the image processing apparatus 100 includes a computer as a basic configuration, and the respective processing units illustrated in FIG. 1 are implemented as the functions of a program running on a processor device.

The image processing unit 121 provides the function of applying an image process designated by an image process 204 to an examination image 200 or a result image obtained by the image process of the immediately preceding round. A program corresponding to each image process is stored in a storage area which is not illustrated, read when carrying out the image process, and carried out. The image processing unit 121 includes a storage area for storing the process object image (such as the examination image 200), and a program work area. The image processing unit 121 outputs a final processing result 206 to the image display apparatus 104. Thus, the image processing unit 121 is also provided with a function related to user interface.

The target area feature quantity extraction unit 111 provides the function of extracting target area feature quantities (size and number of target areas) 202 from the result image obtained by the image process by the image processing unit 121. The target area feature quantity storage unit 112 provides a storage area for storing the extracted target area feature quantities 202. The storage area may include a semiconductor storage device or a hard disk device.

The next image process determination unit 120 provides the function of comparing procedure feature quantities 203 specifying changes in the target area feature quantities 202 between procedures and a past process flow model 205, and of determining the image process 204 to be applied to the process object image next.

(2-2) Display screen example

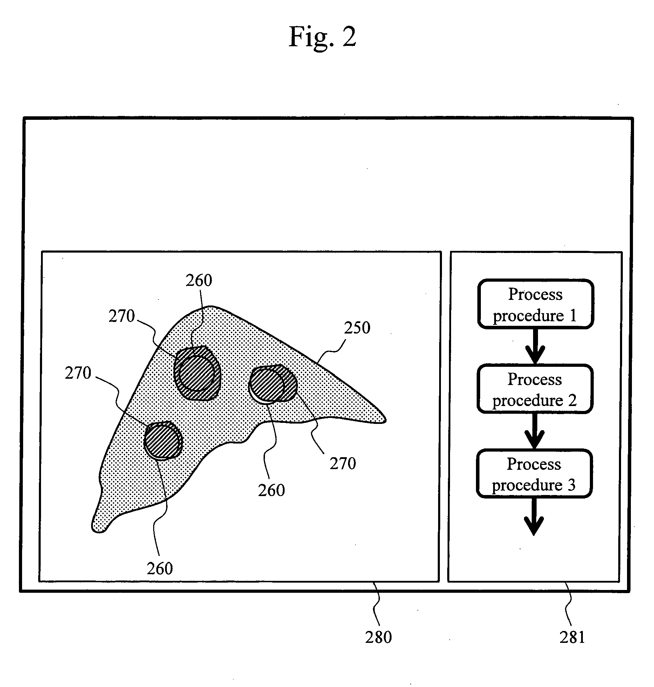

FIG. 2 illustrates a representative display screen displayed on a screen of the image display apparatus 104. The display screen includes an extraction result display screen 280 and a process procedure display screen 281. In the extraction result display screen 280, processing result information is displayed over a contrast enhanced CT image of an organ as a diagnosing object in an overlapping manner. In the case of FIG. 2, a liver CT image 250 is displayed as the contrast enhanced CT image. In the liver CT image 250, a target areas 260 which is a liver cancer affected area, and an extraction result 270 indicating an area extracted by an image process are displayed. The displayed content in the extraction result display screen 280 is updated as the image process proceeds. In the process procedure display screen 281, image process procedures being carried out are displayed. FIG. 2 indicates that the third image process has been completed.

(2-3) Image diagnosis assisting process (automatic target area extraction process)

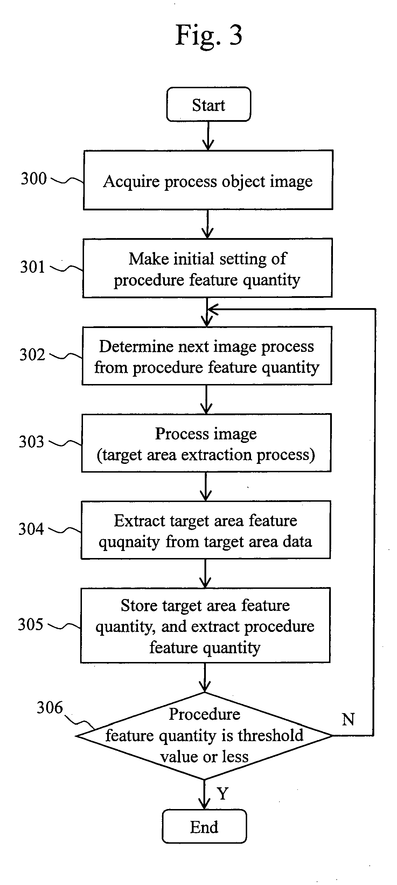

FIG. 3 illustrates the outline of an image diagnosis assisting process carried out by the image processing apparatus 100. In the following description, a case is considered in which the operator wishes to extract liver cancer (such as ischemic liver cancer or hypervascular liver cancer) as the target areas from a contrast enhanced CT image of an examinee. Of course, the target areas are not limited to this and may include any lesion area whose type can be designated from the medical perspective.

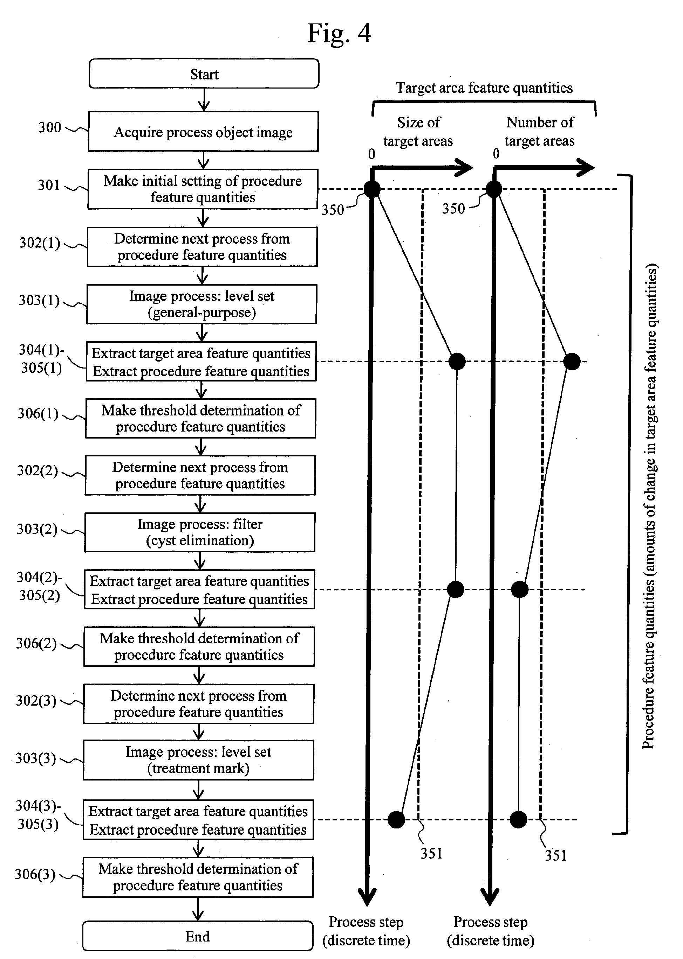

FIG. 4 illustrates temporal changes in the target area feature quantities and the procedure feature quantities (the amounts of change in the target area feature quantities between procedures) acquired in accordance with the progress of the image diagnosis assisting process of FIG. 3. In FIG. 4, the difference in the implemented round of each process is denoted by the numbers in parentheses added at the end. According to the first embodiment, the target area feature quantities are managed in terms of the size and number of the target areas. Thus, in FIG. 4, the changes in the size and number of the target areas as the process proceeds are represented by respective line graphs. In the present specification, the size of the feature quantities of the target areas is specified by volume or area.

In the following, the details of the content of an image diagnosis assisting process carried out by the image processing apparatus 100 according to the first embodiment will be described.

First, a doctor as an operator selects a process object image from the image database 103 (process 300). Specifically, a contrast enhanced CT image is selected.

Then, the doctor makes an initial setting for procedure feature quantities (process 301). The initial setting is the process of determining initial values 350 of the procedure feature quantities, i.e., the size and the number of the target areas. According to the present embodiment, both are initialized to "0".

After the procedure feature quantities are determined, the next image process determination unit 120 carries out a process of determining an image process to be carried out next (process 302 (1)). Because the initial values are "0" in the initial process and there is no amount of change in the procedure feature quantities, the image processing unit 121 is notified of a general-purpose image process (level set algorithm) for ischemic liver cancer extraction. As a result, the image processing unit 121 carries out an extraction process to which the level set algorithm is applied, for example (process 303(1)).

The image processing unit 121 transfers information about areas determined to be target areas 260 based on the processing result of the process to the target area feature quantity extraction unit 111 as target area data 201. The target area feature quantity extraction unit 111 extracts the target area feature quantities (i.e., size and number) contained in the process object image from the given target area data 201 (process 304(1)). The extracted target area feature quantities 202 are stored in the target area feature quantity storage unit 112.

Thereafter, the next image process determination unit 120 searches the target area feature quantity storage unit 112 and extracts the amounts of change in the target area feature quantities (size and number) as procedure feature quantities 203 (process 305(1)).

Next, the next image process determination unit 120 compares the extracted procedure feature quantities 203 with preset threshold values 351 (process 306(1)). When the procedure feature quantities 203 are not more than the threshold values (such as when, in the case of FIG. 4, the procedure feature quantities 203 are not more than threshold values in both size and number), the next image process determination unit 120 notifies the image processing unit 121 of the end of the process. In this case, the image processing unit 121 displays the processing result 206 on the display screen of the image display apparatus 104.

On the other hand, when the procedure feature quantities 203 are not less than the threshold values (such as when, in the case of FIG. 4, the procedure feature quantities 203 exceed the threshold values in both or one of size and number), the next image process determination unit 120 determines the image process to be carried out next based on the procedure feature quantities 203 up to this point in time, and notifies the image processing unit 121 accordingly (process 302(2)). Here, the next image process determination unit 120 searches the process flow model database 102 using the procedure feature quantities 203, and determines an image process of the next round specified with respect to a process flow model with a high similarity degree as the image process to be applied to the image that is the current process object. For example, in the case of FIG. 4, an image filter (cyst removal) is determined as the second image process. In the case of FIG. 4, as the third image process, level set (treatment mark) is determined.

Thus, according to the present embodiment, the processes 302 to 306 are repeatedly carried out until the procedure feature quantities 203 become lower than the predetermined threshold values 351. Namely, as long as a negative result is acquired in the process 306, the process flow with a high degree of similarity with the history of the procedure feature quantities 203 acquired up to the point in time of carrying out each round of the process 302 is extracted from the process flow model database 102, and the image process for the next round which is registered with respect to the process flow model is given as the image process 204 to be applied next by the image processing unit 121.

By carrying out such process, after the initial setting operation by the operator, the image processing apparatus 100 according to the present embodiment can automatically determine an image process until a desired processing result is obtained, and apply the image process to the process object image.

(2-4) Operation for automatic determination of image process

A specific example of the operation of the process carried out when automatically determining the next image process based on the procedure feature quantities 203 will be described.

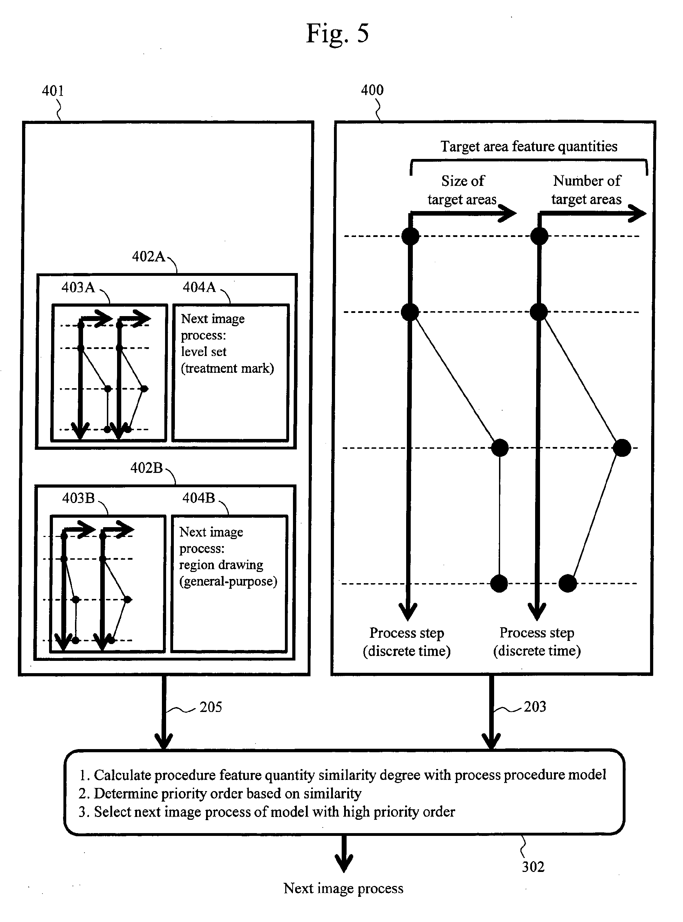

FIG. 5 illustrates the process operation example. FIG. 5 illustrates a specific example 400 of the procedure feature quantities 203 and a specific example 401 of the process flow model 205 stored in the process flow model database 102. In the case of FIG. 5, process flow models 402A and 402B are illustrated.

In the case of FIG. 5, each of the process flow models includes procedure feature quantities 403A or 403B and a next image process 404A or 404B. In the procedure feature quantities 403A and 403B, changes in the size and number of the target areas up to a certain number of implemented rounds are recorded.

Also, in the next image processes 404A and 404B, the content of an image process carried out next to the implemented round corresponding to the procedure feature quantities 403A and 403B is stored. Namely, FIG. 5 illustrates a case where, when process flow models in which five rounds of image processes are carried out exist, for example, a process flow model recording the procedure feature quantities up to the first round and the next image process carried out in the second round, a process flow model recording the procedure feature quantities up to the second round and the next image process carried out in the third round, and similarly a process flow model recording the procedure feature quantities up to each of the subsequent rounds and the next image process carried out in the next round are prepared. In the present example, there is no sixth round of process, so that in the process flow model corresponding to the procedure feature quantities up to the fifth round, "End" is recorded as the next image process.

In this case, the next image process is uniquely determined upon detection of a process procedure model with a high similarity degree with the procedure feature quantities that have appeared with regard to an image currently being processed.

Preferably, a process flow model in which information about the procedure feature quantities for all of the implemented rounds and the image process carried out in each of the rounds may be used. In this case, the procedure feature quantities of the process procedure models may be referenced within the range of rounds of up to the round immediately before the implemented round for which determination is to be made, and, upon detection of a process procedure model with a high similarity degree, the image process carried out in the next implemented round of the detected process procedure model may be read by the next image process determination unit 120.

According to the present embodiment, the next image process determination unit 120 calculates the similarity degree between the process flow model 205 and the procedure feature quantities 203 based on a sum of squared differences of two corresponding procedure feature quantities, for example. In this case, the smaller the sum of squared differences, the higher the similarity degree. Obviously, the similarity degree calculating method is not limited to the sum of squared differences and may include the sum of absolute differences. In the case of FIG. 5, the similarity degree with the specific example 400 is greater for the graph of the process flow model 402A. Thus, the next image process determination unit 120 sets a higher priority for the process flow model 402A with the greater similarity than for the process flow model 402B. Thereafter, the next image process determination unit 120 selects the next image process of the process flow model 402A with higher priority (i.e., level set (treatment mark)) and outputs the next image process to the image processing unit 121.

As described above, by adopting the image processing apparatus 100 according to the first embodiment, when the target areas are to be automatically extracted from the process object image, the operator, after inputting initial conditions, can extract the required target areas from within the process object image without performing any additional operation. Accordingly, the image process content correcting operation by the operator, which is still often required during an image process in conventional apparatuses, can be eliminated. As a result, the operational burden on the operator can be decreased, and the time before the target areas are extracted can be reduced.

(3) Second embodiment

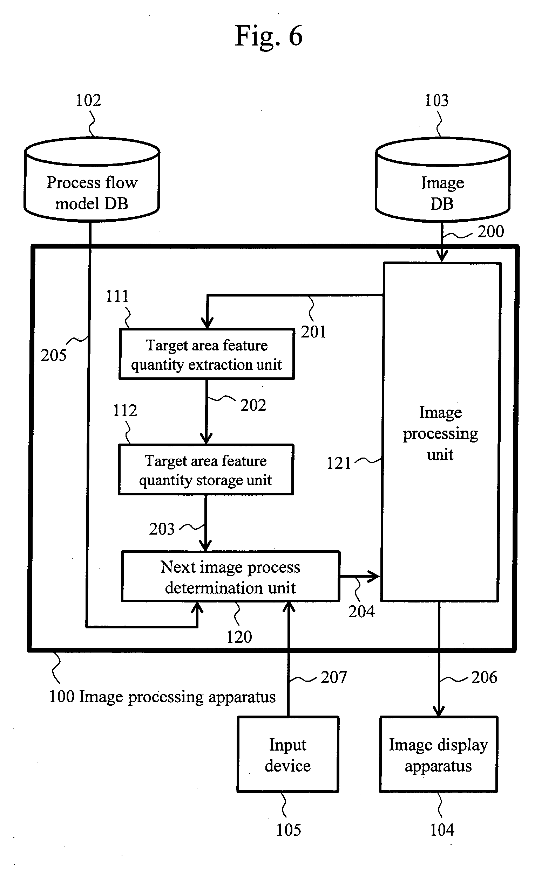

FIG. 6 is a functional block diagram of the image processing system according to the second embodiment. In FIG. 6, parts corresponding to those of FIG. 1 are designated with similar reference signs. The image processing system according to the second embodiment differs from the image processing system according to the first embodiment in that the next image process determination unit 120 is additionally provided with an input device 105 for entering an initial process input 207, and that the next image process determination unit 120 operates with reference to the initial process input 207 inputted by the operator.

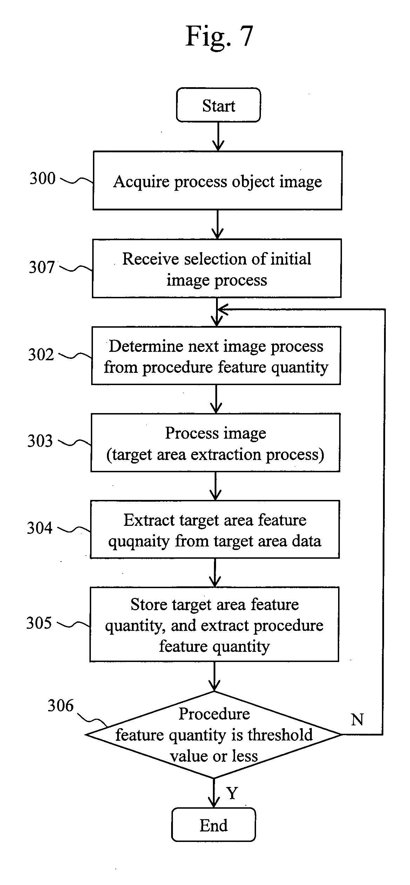

FIG. 7 illustrates the outline of an image diagnosis assisting process carried out by the image processing apparatus 100. In FIG. 7, parts corresponding to those of FIG. 3 are designated with similar reference numerals. As will be seen by comparing FIGS. 7 and 3, according to the present embodiment, a process 307 is carried out instead of the process 301. Namely, the process 307 is carried out after the process 300 and before the process 302.

According to the first embodiment, the initial values of the procedure feature quantities are set by the process 301. In this case, the image process that is carried out in the first round is determined by the set initial values. Obviously, the image process that is carried out in the first round may be modified depending on the initial values given. However, the image process determination is carried out by the next image process determination unit 120, and the operator's intention will not be reflected in the image process determination.

Meanwhile, according to the present embodiment, the operator can specifically select or designate the image process that is carried out in the first round via the input device 105 in the process 307. Preferably, the designation may be carried out prior to the process 300, and the initial process input 207 that is inputted in advance may be taken into the next image process determination unit 120 in the process 307.

According to the present embodiment, the operator can select level set (general-purpose), filter (cyst removal), or level set (treatment mark), for example, as the initial process input 207.

As described above, by adopting the image processing apparatus 100 according to the second embodiment, an image process desired by the operator can be selected or designated as the initial round image process. Thus, an image processing apparatus that can provide an image process in accordance with the operator's intention, in addition to the effect of the first embodiment, can be implemented.

(4) Third embodiment

According to the present embodiment, another process function that may be preferably implemented in the next image process determination unit 120 of the image processing system (FIG. 1) according to the first embodiment will be described. In the case of the present embodiment, the data content of the process flow models stored in the process flow model database 102 also differs from the data content in the first embodiment.

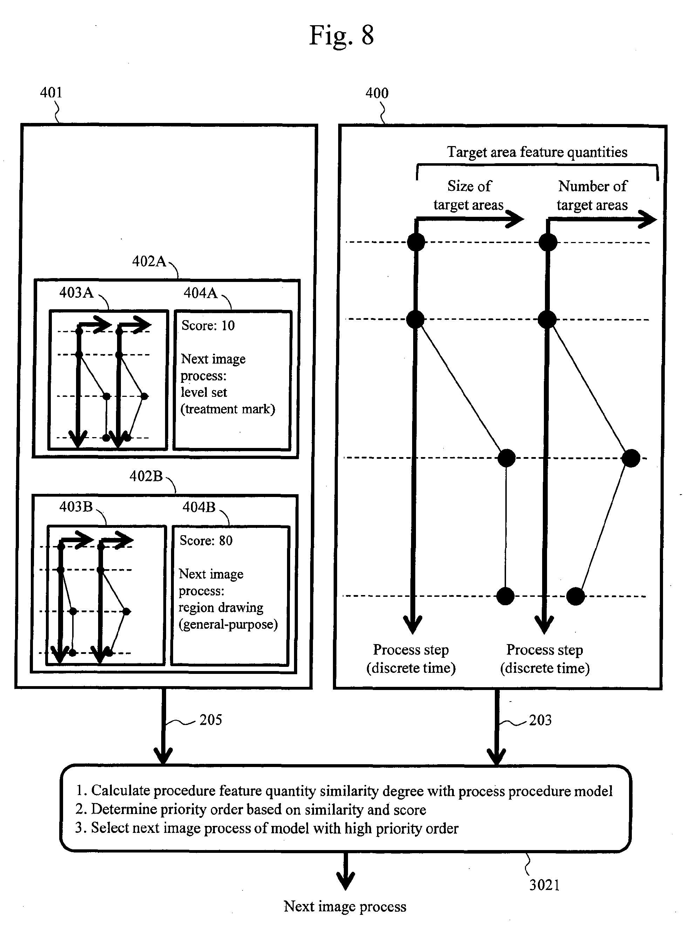

FIG. 8 illustrates the outline of a process carried out by the next image process determination unit 120 according to the present embodiment. In FIG. 8, portions corresponding to those of FIG. 5 are designated with similar reference numerals. According to the present embodiment, in the process flow model database 102, information (score) about the reliability of the data constituting the process flow model is stored as part of the data. The score is used as a correction amount (weight) when the similarity degree of the procedure feature quantities is evaluated. The score is 100 when the reliability is at the highest value (maximum) and zero when at the minimum value. In the case of FIG. 5, the score for the process flow model 402A is "10", while the score for the process flow model 402B is "80".

According to the present embodiment, the next image process determination unit 120 determines the image process to be applied to the process object image next through the following process (process 3021).

First, the next image process determination unit 120 compares the procedure feature quantities 203 acquired with respect to the process object image and the process flow model 205, and calculates the similarity degree between the process flow models 402A and 402B. The similarity degree is an index expressed in ratios: 100% when there is complete agreement, and 0% when there is complete disagreement.

Next, the next image process determination unit 120 determines the priority order of each process flow model by using the reliability and the similarity degree. According to the present embodiment, the reliability and the similarity degree are summed and then standardized by 100 to obtain priority. When the reliability of a process flow model is A1 and its weight is w1, and the similarity degree is A2 and its weight is w2, priority may be calculated by the following expression.

$$\text{Priority} = (w1 \cdot A1 + w2 \cdot A2) / (w1 + w2)$$

If weight w1 = w2 = 1, priority of the process flow model 402A in FIG. 8 is 45 (= (10 + 80) / 2). Meanwhile, priority of the process flow model 402B in FIG. 8 is 60 (= (80 + 40) / 2).

In this case, the priority order is opposite to the priority order of the first embodiment. Namely, the process flow model 402B has the first priority order, and the process flow model 402A has the second priority order. Thus, the next image process determination unit 120 outputs region growing (general-purpose) stored as the next image process 403B of the process flow model 402B to the image processing unit 121.

As described with reference to the present embodiment, by introducing the index indicating the reliability of algorithm with respect to the process flow model as the object of similarity determination, the operator can be presented with an extraction result with higher accuracy than according to the first embodiment.

(5) Fourth embodiment

According to the present embodiment, another process function that may be preferably implemented in the next image process determination unit 120 of the image processing system (FIG. 1) according to the first embodiment will be described.

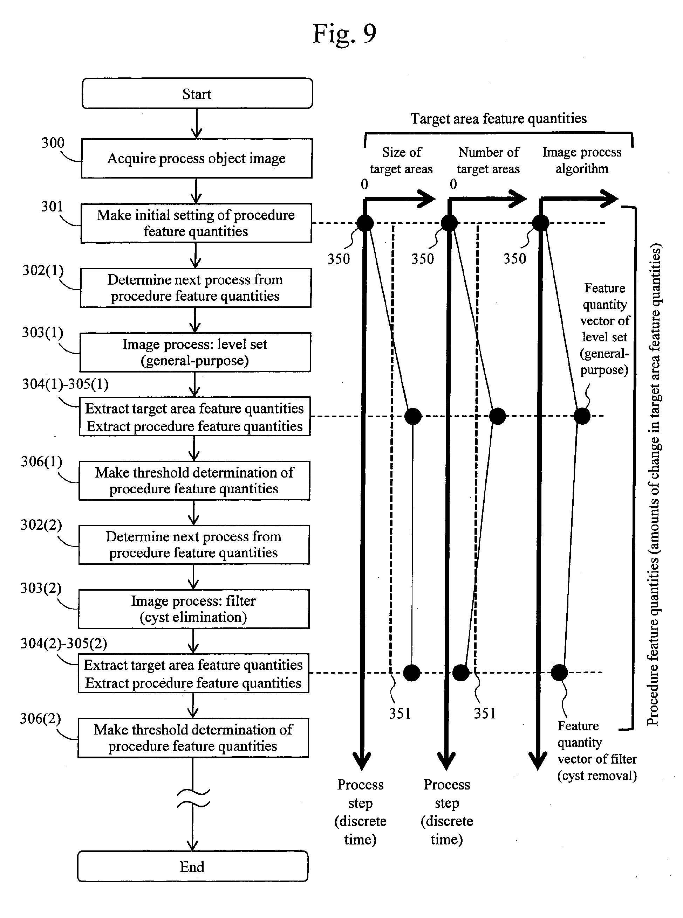

FIG. 9 illustrates a detailed procedure of a process carried out by the next image process determination unit 120 according to the present embodiment. In the case of the present embodiment, the next image process determination unit 120 uses the algorithm of the image process applied in each implemented round as a third procedure feature quantity. Namely, the next image processing unit 120 according to the present embodiment calculates the similarity degree of the process flow models by using the size of target areas, the number of target areas, and the algorithm of the image process to determine a priority order, and outputs the next image process of the process flow model with the highest priority order to the image processing unit 121.

Of course, as a prerequisite, each process flow model stored in the process flow model database 102 includes the image process algorithm in the procedure feature quantities. In the image process algorithm, the parameters used are also stored, in addition to the image process algorithm carried out in each round.

According to the present embodiment, the operator can be provided with a result with high extraction accuracy in which the order of implementation of the image process algorithm is taken into consideration.

(6) Other embodiments

The present invention is not limited to the foregoing embodiments but may include various modifications. For example, the foregoing embodiments have been described in detail to facilitate an understanding of the present invention, and the present invention is not necessarily limited to embodiments having all of the details described. A part of one embodiment may be substituted by a configuration of another embodiment, or a configuration of the other embodiment may be incorporated into a configuration of the one embodiment. With regard to a part of the configuration of an embodiment, additions, deletions, or substitutions may be made.

The configurations, functions, processing units, process means and the like described above may be partly or entirely implemented in the form of hardware, such as an integrated circuit. The configurations, functions and the like described above may be implemented in the form of software, such as a program for implementing the respective functions that is interpreted and executed by a processor. Programs, tables, files, and other information for implementing the respective functions may be stored in a storage device such as a memory, a hard disk, or a solid state drive (SSD), or a storage medium such as an IC card, an SD card, or a DVD.

The illustrated control lines and information lines are only those believed necessary for description purposes, and do not represent all of the control lines or information lines required in a product. It may be considered that, in practice, almost all elements are mutually connected.

REFERENCE SIGNS LIST

| 100 | Image processing apparatus |
| 102 | Process flow model database |
| 103 | Image database |
| 104 | Image display apparatus |
| 105 | Input means device |
| 111 | Target area feature quantity extraction unit |
| 112 | Target area feature quantity storage unit |
| 120 | Next image process determination unit |
| 121 | Image processing unit |
| 200 | Examination image |
| 201 | Target area data |
| 202 | Target area feature quantities |
| 203 | Procedure feature quantities |
| 204 | Image process |
| 205 | Process flow model |
| 206 | Processing result |
| 207 | Initial process input |
| 250 | Liver CT image |
| 260 | Target areas |
| 270 | Extraction result of target areas |
| 280 | Extraction result display screen |
| 281 | Process procedure display screen |
| 350 | Initial values |
| 351 | Threshold values |

**Claims**

1. An image processing apparatus for extracting a target area from a process object image by applying a plurality of image processes, the target area being a region different from a peripheral area in the process object image, the image processing apparatus comprising:

    a processing unit that stores a first history regarding a processing result of an application of a first image process procedure to the process object image;
    a processing unit that reads, from a database accumulating a candidate for an image process to be applied after the first image process procedure, and a second history regarding a processing result of an application of a second image process procedure corresponding to the candidate, the second history, that evaluates a similarity degree between the first history and the second history, and that determines an image process corresponding to the second history with a high evaluation result as a next candidate; and
    a processing unit that carries out a target area extraction process based on the determined image process.

2. The image processing apparatus according to claim 1, wherein the histories include information about a change in a feature quantity regarding the target area extracted in each of one or more image processes.

3. The image processing apparatus according to claim 2, wherein the feature quantity includes a number or a size of the target area.

4. The image processing apparatus according to claim 2, wherein the feature quantity includes information about an image process algorithm.

5. The image processing apparatus according to any one of claims 2 to 4, wherein the process object image is a medical image, and the target area is a lesion area.

6. The image processing apparatus according to claim 1, wherein:

   the database stores an evaluation index associated with the second image process procedure; and
   the evaluation of the similarity degree between the first history and the second history includes evaluating the similarity degree with reference also to the evaluation index.

7. An image processing method carried out in a computer for extracting a target area from a process object image by applying a plurality of image processes, the target area being a region different from a peripheral area in the process object image,
   the image processing method comprising:

   a process of storing a first history regarding a processing result of an application of a first image process procedure to the process object image;
   a process of reading, from a database accumulating a candidate for an image process applied after the first image process procedure, and a second history regarding a processing result of an application of a second image process procedure corresponding to the candidate, the second history, evaluating a similarity degree between the first history and the second history, and determining an image process corresponding to the second history with a high evaluation result as a next candidate; and
   a process of carrying out a target area extraction process based on the determined image process.

8. The image processing method according to claim 7, wherein the histories include information about a change in a feature quantity regarding the target area extracted in each of one or more image processes.

9. The image processing method according to claim 8, wherein the feature quantity includes a number or a size of the target area.

10. The image processing method according to claim 8, wherein the feature quantity includes information about the image process algorithm.

11. The image processing method according to any one of claims 8 to 10, wherein the process object image is a medical image, and the target area is a lesion part.

12. The image processing method according to claim 7, wherein:

    the database stores an evaluation index associated with the second image process procedure; and
    the evaluating the similarity degree between the first history and the second history includes evaluating the similarity degree with reference also to the evaluation index.

# Fig. 1

Fig. 2

# Fig. 3

```
                        ┌──────────────┐
                        │    Start     │
                        └──────┬───────┘
                               │
                               ▼
            ┌──────────────────────────────────────┐
   300 ─────│      Acquire process object image     │
            └──────────────────┬───────────────────┘
                               │
                               ▼
            ┌──────────────────────────────────────┐
   301 ─────│         Make initial setting of       │
            │        procedure feature quantity     │
            └──────────────────┬───────────────────┘
                               │
                               ▼◄────────────────────┐
            ┌──────────────────────────────────────┐ │
   302 ─────│     Determine next image process      │ │
            │    from procedure feature quantity    │ │
            └──────────────────┬───────────────────┘ │
                               │                      │
                               ▼                      │
            ┌──────────────────────────────────────┐ │
   303 ─────│            Process image               │ │
            │      (target area extraction process) │ │
            └──────────────────┬───────────────────┘ │
                               │                      │
                               ▼                      │
            ┌──────────────────────────────────────┐ │
   304 ─────│        Extract target area feature    │ │
            │       quqnaity from target area data  │ │
            └──────────────────┬───────────────────┘ │
                               │                      │
                               ▼                      │
            ┌──────────────────────────────────────┐ │
            │        Store target area feature      │ │
   305 ─────│     quantity, and extract procedure   │ │
            │             feature quantity          │ │
            └──────────────────┬───────────────────┘ │
                               │                      │
                               ▼                      │
                          ╱─────────╲                 │
   306              ╱    Procedure    ╲      N        │
                ╱  feature quantity is threshold ╲────┘
                ╲      value or less         ╱
                     ╲─────────╱
                          │ Y
                          ▼
                   ┌──────────────┐
                   │     End      │
                   └──────────────┘
```

# Fig. 4

# Fig. 5

401

400

**Target area feature quantities**

402A

403A          404A

Next image
process:
level set
(treatment mark)

Size of
target areas

Number of
target areas

402B

403B          404B

Next image
process:
region drawing
(general-purpose)

Process step
(discrete time)

Process step
(discrete time)

205

203

1. Calculate procedure feature quantity similarity degree with process procedure model
2. Determine priority order based on similarity
3. Select next image process of model with high priority order

302

Next image process

# Fig. 6

# Fig. 7

```
                    ┌──────────┐
                    │  Start   │
                    └────┬─────┘
                         │
                         ▼
300  ~~  ┌──────────────────────────────┐
         │   Acquire process object image│
         └──────────────┬───────────────┘
                        │
                        ▼
307  ~~  ┌──────────────────────────────┐
         │   Receive selection of initial│
         │        image process         │
         └──────────────┬───────────────┘
                        │
                        ▼
302  ~~  ┌──────────────────────────────┐
         │  Determine next image process │
         │  from procedure feature quantity│
         └──────────────┬───────────────┘
                        │
                        ▼
303  ~~  ┌──────────────────────────────┐
         │        Process image          │
         │  (target area extraction process)│
         └──────────────┬───────────────┘
                        │
                        ▼
304  ~~  ┌──────────────────────────────┐
         │    Extract target area feature │
         │   quqnaity from target area data│
         └──────────────┬───────────────┘
                        │
                        ▼
305  ~~  ┌──────────────────────────────┐
         │    Store target area feature  │
         │  quantity, and extract procedure│
         │       feature quantity        │
         └──────────────┬───────────────┘
                        │
                        ▼
306  ~~      ╱ Procedure           ╲    N
            ╱ feature quantity is threshold ╲────►
            ╲     value or less    ╱
             ╲                    ╱
                    │ Y
                    ▼
              ┌──────────┐
              │   End    │
              └──────────┘
```

# Fig. 8

401

402A

403A      404A

Score: 10

Next image
process:
level set
(treatment mark)

402B

403B      404B

Score: 80

Next image
process:
region drawing
(general-purpose)

400

Target area feature quantities

Size of
target areas

Number of
target areas

Process step
(discrete time)

Process step
(discrete time)

205

203

1. Calculate procedure feature quantity similarity degree with process procedure model
2. Determine priority order based on similarity and score
3. Select next image process of model with high priority order

3021

Next image process

# Fig. 9

**INTERNATIONAL SEARCH REPORT**

| International application No. |
|---|
| PCT/JP2012/062892 |

A. CLASSIFICATION OF SUBJECT MATTER
*A61B6/03*(2006.01)i, *A61B6/00*(2006.01)i, *G06T1/00*(2006.01)i

According to International Patent Classification (IPC) or to both national classification and IPC

B. FIELDS SEARCHED

Minimum documentation searched (classification system followed by classification symbols)
A61B6/03, A61B6/00, G06T1/00

Documentation searched other than minimum documentation to the extent that such documents are included in the fields searched
Jitsuyo Shinan Koho          1922-1996   Jitsuyo Shinan Toroku Koho   1996-2012
Kokai Jitsuyo Shinan Koho    1971-2012   Toroku Jitsuyo Shinan Koho   1994-2012

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2005-270318 A  (Konica Minolta Medical & Graphic, Inc.), 06 October 2005 (06.10.2005), entire text; all drawings (Family: none) | 1-12 |
| A | JP 2005-230456 A  (President of National Center of Neurology and Psychiatry, Eisai Co., Ltd., Dainippon Printing Co., Ltd.), 02 September 2005 (02.09.2005), entire text; all drawings (Family: none) | 1-12 |
| A | JP 2001-094829 A  (Canon Inc.), 06 April 2001 (06.04.2001), entire text; all drawings & US 2005/0036670 A1 | 1-12 |

☒   Further documents are listed in the continuation of Box C.          ☐   See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 31 May, 2012 (31.05.12) | 12 June, 2012 (12.06.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| | International application No. |
|---|---|
| | PCT/JP2012/062892 |

C (Continuation).    DOCUMENTS CONSIDERED TO BE RELEVANT

| Category* | Citation of document, with indication, where appropriate, of the relevant passages | Relevant to claim No. |
|---|---|---|
| A | JP 8-044851 A  (Hitachi Medical Corp.), 16 February 1996 (16.02.1996), entire text; all drawings (Family: none) | 1-12 |

Form PCT/ISA/210 (continuation of second sheet) (July 2009)

**EP 2 716 225 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009082452 A